# EUROPÄISCHE PATENTSCHRIFT

(11) **EP 2 181 450 B1**
(45) Veröffentlichungstag und Bekanntmachung des Hinweises auf die Patenterteilung: **19.06.2013**
(21) Anmeldenummer: 08784948.5
(22) Anmeldetag: 22.07.2008
(51) Int. Cl.: G21K 5/04, A61N 5/10

(54) **SCHNELLE REGELUNG DER REICHWEITE VON HOCHENERGETISCHEN IONENSTRAHLEN FÜR PRÄZISIONSBESTRAHLUNGEN VON BEWEGTEN ZIELVOLUMINA**
QUICK REGULATION OF THE RANGE OF HIGH-ENERGY ION BEAMS FOR PRECISION IRRADIATION OF MOVING TARGET VOLUMES
RÉGULATION RAPIDE DE LA PORTÉE DE FAISCEAUX IONIQUES À HAUTE ÉNERGIE POUR L'IRRADIATION DE PRÉCISION DE VOLUMES CIBLES EN MOUVEMENT

(30) Priorität: 24.08.2007 DE 102007040299; 15.11.2007 DE 102007054919
(43) Veröffentlichungstag der Anmeldung: 05.05.2010
(73) Patentinhaber: GSI Helmholtzzentrum für Schwerionenforschung GmbH, 64291 Darmstadt (DE)
(72) Erfinder: SCHARDT, Dieter, 64291 Darmstadt (DE); BERT, Christoph, 63741 Aschaffenburg (DE); SAITO, Nami, 64291 Darmstadt (DE); FRANCZAK, Bernhard, 64293 Darmstadt (DE); CHAUDHRI, Naved, 69123 Heidelberg (DE); PLESKAC, Radek, 64291 Darmstadt (DE)
(74) Vertreter: Blumbach Zinngrebe
(86) Internationale Anmeldenummer: PCT/EP2008/005984
(87) Internationale Veröffentlichungsnummer: WO 2009/026997

(56) Entgegenhaltungen:
- WO-A-2005/110495
- DE-A1-102005 053 971
- JP-A- 2006 341 010
- SVEN OLIVER GRÖZINGER ET AL: "Simulations to design an online motion compensation system for scanned particle beams" PHYSICS IN MEDICINE AND BIOLOGY, TAYLOR AND FRANCIS LTD. LONDON, GB, Bd. 51, Nr. 14, 21. Juli 2006 (2006-07-21), Seiten 3517-3531, XP020095856 ISSN: 0031-9155

## Beschreibung

### Beschreibung der Erfindung

Die Erfindung betrifft eine Vorrichtung und ein Verfahren zum Anpassen der Reichweite eines Ionenstrahls, insbesondere für die therapeutische Bestrahlung.

### Hintergrund der Erfindung

Im Rahmen des Pilotprojekts "Tumortherapie mit schweren Ionen" werden bei der Gesellschaft für Schwerionenforschung seit 1997 Krebspatienten mit lokalisierten Tumoren im Bereich von Kopf, Hals und Becken mit Kohlenstoffionen in einem Energiebereich von 80 bis 430 MeV/u bestrahlt.

Das intensitätsgesteuerte Rasterscan-Verfahren, bei dem ein feiner Ionenstrahl rasterförmig schichtweise über das Zielvolumen geführt wird, ermöglicht eine hochkonformale, d.h. an die Form des Tumors angepasste, und hochwirksame Bestrahlung von tief im Gewebe liegenden Tumoren bei gleichzeitiger maximaler Schonung des umliegenden gesunden Gewebes.

Eine präzise 3D-Dosisapplikation ist aber bei einer konstant vorgegebenen kinetischen Energie des Ionenstrahls nur dann gegeben, wenn sich die Lage des Zielvolumens zeitlich nicht verändert. Das Zielvolumen wird oft auch als "clinical target volume (CTV) bezeichnet. Im Bereich des Kopfes kann dies durch Immobilisation mittels individuell gefertigter Kopfmasken erreicht werden. Bei inneren Organen, die sich beispielsweise infolge der Atmung bewegen, wie zum Beispiel die Lunge oder Organe im Thoraxbereich, ist dies jedoch nicht möglich. Beispielsweise ist im Brustbereich eine Bewegung des Zielvolumens besonders problematisch, da das Zielvolumen in den "Schattenbereich" einer Rippe bewegt werden kann.

Die vorliegende Erfindung befasst sich mit der Anpassung der Eindringtiefe oder Reichweite des Ionenstrahls, d.h. der Position des Bragg-Maximums im bestrahlten Gewebe, vorzugsweise bei bewegten Zielvolumina. Während, in Strahlrichtung gesehen, seitliche Verschiebungen des Zielvolumens mittels einer schnellen Ansteuerung von Rasterscan-Magneten kompensiert werden können, erfordern Verschiebungen in Strahlrichtung eine schnelle Anpassung der spezifischen Energie der Ionen und damit der Position des Bragg-Maximums in der Tiefe des Gewebes.

Dies wird mit Hilfe einer passiven sogenannten Reichweitenmodulation erreicht. Ein feiner Ionenstrahl hoher Energie, von etwa 50 bis 400 MeV/u, und scharf definierter Energie, wie er von einem Synchrotron oder einem Zyklotronbeschleuniger geliefert wird, erfährt beim Durchgang durch ein Stück homogener Materie der Dicke d einen wohldefinierten Energieverlust. Durch Variation der Dicke d dieses passiven Reichweitenmodulators, welcher auch als "Range shifter" bezeichnet wird, lässt sich die Ausgangsgeschwindigkeit der Ionen und damit deren Reichweite im Gewebe einstellen. Die Variation der Dicke wird durch eine keilförmige, stufenförmige oder gekrümmte Struktur des Reichweitenmodulators erreicht.

Eine solche Lösung ist bereits in der Anmeldung WO 2005/120641 beschrieben, wobei dort der Reichweitenmodulator aus zwei gegeneinander verschiebbaren Keilen besteht. Diese sind auf einer elektromotorisch angetriebenen Linearachse montiert und befinden sich unmittelbar vor dem Patienten.

Da die Reichweitenmodulatoren des Standes der Technik im Allgemeinen beträchtliche Massen aufweisen, reagieren sie zum einen nicht hinreichend schnell und zum anderen sind entsprechend hohe mechanische und daher kosten- und fertigungsintensive Anforderungen an die Linearachsenantriebe zu stellen. Weiterhin führt die schnelle Bewegung der Keilantriebe zu erheblichen Fahrgeräuschen, die für Patienten unangenehm werden können.

Da sich der Reichweitenmodulator aus dem Stand der Technik zudem unmittelbar vor dem Patienten befindet, besitzen die Ionenstrahlen aufgrund des statistisch verteilten Energieverlusts keine präzise kinetische Energie. Als problematischer erweist sich jedoch die durch eine transversale Vielfachstreuung bedingte Aufweitung des Ionenstrahls, so dass bei dem bekannten System der Reichweitenmodulator in einem möglichst geringen Abstand (typischerweise ca. 10 cm) vor dem Patienten positioniert werden muss. Zudem können sekundäre Fragmente, wie Neutronen, die infolge von Kernreaktionen in einem Reichweitenmodulator erzeugt werden, nicht mehr vom Ionenstrahl getrennt werden und erzeugen eine unkontrollierte und unerwünschte zusätzliche Dosis.

SVEN OLIVER GRÖZINGER ET AL: "Simulations to design an online motion compensation system for scanned particle beams", PHYSICS IN MEDICINE AND BIOLOGY, TAYLOR AND FRANCIS LTD. LONDON, GB, Bd. 51, Nr. 14, 21. Juli 2006 (2006-07-21), Seiten 3517-3531, XP020095856, ISSN: 0031-9155, offenbart eines Systems zur Echtzeit-Bewegungskompensation in der Partikelstrahltherapie und nennt als wahrscheinlichste technische Realisation zur "longitudinalen" Kompensation einen schenllen passiven Energiemodulator, z.B. ein Keilsystem.

JP 2006 341010 A (MITSUBISHI ELECTRIC CORP) 21. Dezember 2006 (2006-12-21) offenbart eine Teilchenstrahl-Therapieanlage, die es u.a. erlaubt, die Verfahrgeräusche und die Einstellzeit erheblich zu reduzieren.

### Allgemeine Beschreibung der Erfindung

vor diesem Hintergrund hat sich die vorliegende Erfindung daher zur Aufgabe gestellt, eine Vorrichtung und ein Verfahren zum Anpassen der Reichweite eines Ionenstrahls an ein Zielvolumen, beispielsweise für die therapeutische Bestrahlung, bereitzustellen, welche die Nachteile des Standes der Technik wenigstens reduzieren.

Hierbei soll insbesondere auch eine gegenüber dem Stand der Technik schnellere Anpassung der Reichweite der Ionen an die Bewegung des Zielvolumens möglich sein.

Gelöst wird diese Aufgabe bereits durch die Vorrichtung zur Kompensation der Bewegung des zielvolumens in Strahlrichtung und das Verfahren zum Regeln eines Ionenstrahls, insbesondere für die therapeutische Bestrahlung, gemäß den Merkmalen der beiden unabhängigen Ansprüche. Vorteilhafte Ausführungsformen sind Gegenstand der jeweiligen Unteransprüche.

Die Erfindung betrifft eine Vorrichtung zur Kompensation der Bewegung eines Zielvolumens in Strahlrichtung bei der therapetutischen Behandlung eines Patienten mittels Bestrahlung des Zielvolumens durch Ionen, indem die Reichweite eines Ionenstrahls an die Bewequng des zielvolumens angepasst wird, umfassend
- eine Reichweitenanpassungseinrichtung für den Ionenstrahl, in welcher der Ionenstrahl beim Durchqueren unterschiedlicher Bereiche der Reichweitenanpassungseinrichtung einen unterschiedlichen Energieverlust erfährt,
- einen Sensor zur Erfassung der Position des zielvolumens, welcher eine die Position beschreibende Information bereitstellt,
- eine Regeleinrichtung, welche die Information des Sensors empfängt und ein zur Position des Zielvolumens korreliertes Steuersignal erzeugt und
- eine steuerbare erste Ablenkeinrichtung, an die das Steuersignal geschickt wird und welche in Strahlrichtung vor der Reichweitenanpassungseinrichtung angeordnet ist, zum Ablenken des Ionenstrahls aus seiner ursprünglichen Strahlachse auf eine zur ursprünglichen Strahlachse geneigte Strahlachse, so dass der Ionenstrahl auf unterschiedliche Bereiche der Reichweitenanpassungseinrichtung gelenkt wird, wobei die Reichweitenanpassungseinrichtung auf der geneigten Strahlachse angeordnet ist, wobei die Ablenkung des Ionenstrahls in Ansprechen auf das Steuersignal mittels der ersten Ablenkeinrichtung derart angepasst wird, dass der Ionenstrahl einen entsprechenden Bereich der Reichweitenanpassungseinrichtung durchläuft, so dass die Reichweite des Ionenstrahls auf die durch die Bewegung des Zielvolumens in Strahlrichtung veränderte Position des Zielvolumens abgestimmt ist und das Bragg-Maximum in den Bereich des zielvolumens geschoben wird, derart dass dadurch die Bewegung des zielvolumens in Strahlrichtung kompensiert wird, und
- eine steuerbare zweite Ablenkeinrichtung zum Ablenken des Ionenstrahis, welche in Strahlrichtung nach der Reichweitenanpassungseinrichtung angeordnet ist, so dass der Ionenstrahl aus der geneigten Strahlachse heraus auf eine Strahlachse gelenkt wird, welche parallel und seitlich versetzt zur ursprünglichen Strahlachse, auf welcher der Ionenstrahl in die erste Ablenkeinrichtung eintritt, verläuft.

Weiterhin beansprucht die vorliegende Erfindung ein Verfahren zur Bestrahlung eines Phantomtargets zur Bestimmung und/oder Verifikation von Verfahrensparametern, umfassend die folgenden Verfahrensschritte:
- Bereitstellen des Ionenstrahis,
- Feststellen einer Referenzposition eines zu bestrahlenden Zielvolumens eines Phantomtargets,
- Einstellen der Reichweite des Ionenstrahls derart, dass diese an die Referenzposition des Zielvolumens angepasst wird, so dass das Bragg-Maximum im Bereich des Zielvolumens liegt,
- Nachweisen einer Änderung der Referenzposition durch Bewegung des Zielvolumens,
- Ablenken des Ionenstrahls durch Einstellen eines angelegten Feldes einer ersten Ablenkeinrichtung aus seiner ursprünglichen Strahlachse auf eine zur ursprünglichen Strahlachse geneigten Strahlachse, so dass der Ionenstrahl auf eine Stelle einer Reichweitenanpassungseinrichtung, welche in Strahlrichtung vor dem Zielvolumen angeordnet ist, geführt wird, so dass der Ionenstrahl beim Durchlaufen der Reichweitenanpassungseinrichtung an dieser Stelle einen Energieverlust erfährt, welcher derart angepasst ist, dass die Positionsänderung des Zielvolumens durch die Anpassung der Reichweite des Ionenstrahis, indem das Bragg-Maximum in den Bereich des Zielvolumens geschoben wird, kompensiert wird,
wobei die Reichweitenanpassungseinrichtung auf der geneigten Strahlachse angeordnet ist,
wobei der Ionenstrahl nach der Reichweitenanpassung aus der geneigten Strahlachse heraus auf eine Strahlachse gelenkt wird, welche parallel und seitlich versetzt zur ursprünglichen Strahlachse, auf welcher der Ionenstrahl in die erste Ablenkeinrichtung eintritt, verläuft.

Die Vorrichtung ist insbesondere ausgebildet zum Ausführen des erfindungsgemäßen Verfahrens. Das Verfahren ist insbesondere ausführbar mittels der erfindungsgemäßen Vorrichtung. Sowohl das erfindungsgemäße Verfahren zum Regeln eines Ionenstrahls als auch die erfindungsgemäße Vorrichtung zum Anpassen der Reichweite eines Ionenstrahls sind geeignet für die therapeutische Bestrahlung. Eine weitere potentielle Anwendung ist die Bestrahlung eines Phantomtargets (z.B. Wasserphantom) zur Bestimmung und verifikation von Verfahrensparametern. Eine solche Bestimmung und Verifikation von Verfahrensparametern wird beispielsweise vor und/oder nach einer eigentlichen Bestrahlung durchgeführt. Daher beinhaltet die vorliegende Erfindung auch eine Vorrichtung zur Bestimmung und/oder Verifikation von Verfahrensparametern. Weiterhin beinhaltet die vorliegende Erfindung auch ein verfahren zur Bestimmung und/oder Verifikation von verfahrensparametern.

Der Begriff Ionenstrahlen umfasst schwere geladene Teilchen, wie Protonen, Kohlenstoffionen, Sauerstoffionen und/oder auch andere, insbesondere zur therapeutischen Anwendung, geeignete Teilchen wie z.B. Pionen, Anti-Protonen oder auch Verbindungen der genannten Teilchen. Der Ionenstrahl wird vor der Reichweitenanpassung mittels der ersten Ablenkeinrichtung aus seiner ursprünglichen Strahlachse heraus gelenkt. vorzugsweise wird der Ionenstrahl von der ersten Ablenkeinrichtung nur in einer Ebene abgelenkt.

Die Ablenkung des Ionenstrahls wird durch ein angelegtes, vorzugsweise magnetisches Feld, welches mittels der ersten Ablenkeinrichtung erzeugt wird, bewirkt.

Der Ionenstrahl durchdringt, aufgrund seiner veränderten Strahlachse, die Reichweitenanpassungseinrichtung in unterschiedlichen Bereichen. In Abhängigkeit von dem durchdrungenen Bereich oder dem Auftreffort des Ionenstrahls auf der Reichweitenanpassungseinrichtung erfährt der Ionenstrahl einen unterschiedlichen Energieverlust.

Da der Ionenstrahl, in Abhängigkeit von seinem Auftreffort auf der Reichweitenanpassungseinrichtung, beim Durchqueren der Reichweitenanpassungseinrichtung einen unterschiedlichen Energieverlust erfährt, wird die Reichweitenanpassungoeiarichtung auch als ortsabhängiges Energieverlustelement bezeichnet. In der vorliegenden Beschreibung wird die Reichweitenanpassungseinrichtung zudem auch als Reichweitenmodulator bezeichnet.

Um den unterschiedlichen Energieverlust bewirken zu können, bzw. um die Ionenstrahlen unterschiedlicher Reichweite oder unterschiedlicher Energie bereitstellen zu können, weist die Reichweitenanpassungseinrichtung in einer Ausführungsform eine unterschiedliche Dicke in zumindest einer zur Strahlachse senkrechten Dimension auf. Entsprechend wird der Ionenstrahl von der ersten Ablenkeinrichtung in einer Ebene senkrecht zur Strahlachse so abgelenkt, dass der Ionenstrahl die Reichweitenanpassungseinrichtung an Stellen unterschiedlicher Dicke durchdringt.

Der Energieverlust eines Ionenstrahls ist jedoch nicht nur von der Dicke, sondern auch von dem Material oder der Dichte der Reichweitenanpassungseinrichtung abhängig. Daher wird in einer ergänzenden oder alternativen Ausführungsform der unterschiedliche Energieverlust des Ionenstrahls durch eine Variation der materiellen Zusammensetzung der Reichweitenanpassungseinrichtung erzeugt. Zum Beispiel können hierzu einzelne Bereiche der Reichweitenanpassungseinrichtung, vorzugsweise in zumindest einer zur Strahlachse senkrechten Dimension, gezielt mit anderen Materialien angereichert werden.

Die Ablenkeinrichtung richtet den Ionenstrahl gezielt auf eine gewünschte Stelle auf der Reichweitenanpassungseinrichtung. Vorzugsweise wird der Ionenstrahl nur in einer Dimension, insbesondere entlang einer Geraden, über die Reichweitenanpassungseinrichtung bewegt. Da der Ionenstrahl selbst über die Reichweitenanpassungseinrichtung bewegt wird, liegt somit eine aktive Reichweitenanpassung oder eine aktive Reichweitenmodulation vor. Die Reichweitenanpassungseinrichtung selbst ist dagegen statisch ausgebildet und wird somit nicht bewegt. Es erfolgt ein Verändern der Strahlachse relativ zu einem festen Bezugspunkt.

Die Reichweitenanpassungseinrichtung befindet sich erfindungsgemäß und im Unterschied zum Stand der Technik nicht unmittelbar vor dem Zielvolumen. Daher können bei entsprechender Fokussierung des Ionenstrahls die Abmessungen der Reichweitenanpassungseinrichtung klein gehalten werden. Die Reichweitenanpassungseinrichtung weist in ihrer Ausdehnung quer zur Strahlachse eine Länge von 2 cm bis 10 cm und eine Breite von 0,5 cm bis 5 cm auf. Die Länge bezieht sich auf die Ebene, in welcher der Ionenstrahl vor dem Auftreffen auf der Reichweitenanpassungseinrichtung abgelenkt wird. Die Breite steht senkrecht dazu. In einer Ausführungsform ist die Reichweitenanpassungseinrichtung ein Festkörper.

Die vorliegende Erfindung umfasst eine steuerbare zweite Ablenkeinrichtung zum Ablenken des Ionenstrahls. In einer bevorzugten Ausführungsform erzeugt die zweite Ablenkeinrichtung ein magnetisches Feld zum Ablenken des Ionenstrahls. Die zweite Ablenkeinrichtung ist in Strahlrichtung gesehen nach der Reichweitenanpassungseinrichtung positioniert. Mittels dieser zweiten Ablenkeinrichtung wird die Strahlachse des Ionenstrahls nach dem Anpassen der Reichweite bzw. nach dem Passieren der Reichweitenanpassungseinrichtung verändert oder angepasst, so dass der Ionenstrahl angepasster Reichweite auf das Zielvolumen gelenkt werden kann.

Die Regeleinrichtung, welche die Information des Sensors über die Position des zielvolumens empfängt, erzeugt ein weiteres, zur Position des Zielvolumens und zur angepassten Reichweite des Ionenstrahls, korreliertes Steuersignal. Die zweite Ablenkeinrichtung erhält dieses, zur Position des Zielvolumens, korrelierte Steuersignal zum Ablenken des Ionenstrahls. Die Ablenkung des Ionenstrahls durch die zweite Ablenkeinrichtung ist in Ansprechen auf das Steuersignal derart angepasst, dass die Reichweite bzw. die kinetische Energie des Ionenstrahls, der auf die ursprüngliche Achse zurückgeführt wird, auf die Position bzw. Tiefe des Zielvolumens im Körper abgestimmt ist und das Bragg-Maximum in den Bereich des Zielvolumens geschoben wird.

Der Ionenstrahl wird nach der Reichweitenanpassung und vor dem Auftreffen auf dem Zielvolumen wenigstens einmal in zumindest einer zur Strahlachse lateralen Dimension kollimiert. Dazu ist ein Kollimator in Strahlrichtung gesehen nach der zweiten Ablenkeinrichtung positioniert. Der Kollimator bewirkt eine Begrenzung des Ionenstrahls in zumindest einer Dimension senkrecht zur Strahlachse. Vorzugsweise erfolgt die Begrenzung in der Ebene, in der auch die vorige Ablenkung durch die erste bzw. zweite Ablenkeinrichtung erfolgte.

Durch die zweite Ablenkeinrichtung und den Kollimator wird ein Impulsfilter gebildet. Dadurch werden Anteile des Ionenstrahls, welche eine nicht an die Lage des Zielvolumens angepasste Reichweite aufweisen, aus dem Ionenstrahl gefiltert oder entfernt.

Eine Rasterscan-Einrichtung zum zweidimensionalen Scannen des Ionenstrahls in der Ebene senkrecht zur Strahlachse ist zur Veränderung der lateralen Lage des Ionenstrahls, insbesondere zum Abrastern des Zielvolumens, im Strahlengang angeordnet. Die Rasterscan-Einrichtung ist in Strahlrichtung nach dem Kollimator positioniert. Sie befindet sich vorzugsweise etwa 5 m bis etwa 6 m vor dem Zielvolumen. Sie befindet sich in einem Bereich, in dem keine Mittel zur Strahlführung mehr vorhanden sind. Folglich ist auch die Reichweitenanpassungseinrichtung in Strahlrichtung vor der Rasterscan-Einrichtung angeordnet. Sofern sich das Zielvolumen bewegt und sich somit die Position des Zielvolumens verändert, kann mittels der Rasterscan-Einrichtung eine laterale Positionsänderung des Zielvolumens kompensiert werden. In Verbindung mit der vorstehend beschriebenen Anpassung der Reichweite des Ionenstrahls kann somit insgesamt eine dreidimensionale Positionsänderung des Zielvolumens, insbesondere in Echtzeit, d.h. während des Vorgangs der Bestrahlung, kompensiert werden.

Da ein zu bestrahlendes Zielvolumen im Allgemeinen sowohl eine bestimmte laterale Ausdehnung als auch eine bestimmte Ausdehnung in der Tiefe aufweist, ist auch bei nichtbewegten Zielvolumina eine Verändern sowohl der lateralen Position als auch der Tiefenposition erforderlich, um alle Bereiche des Zielvolumens mit der entsprechenden maximalen Energiedeposition bzw. Schädigung erfassen oder abrastern zu können. Dazu wird ein sogenannter Bestrahlungsplan erstellt. Das Zielvolumen wird dann sowohl in lateraler Lage, mittels der Rastercan-Magnete, als auch in der Tiefenlage, mittels der Reichweitenanpassungseinrichtung, gemäß dem Bestrahlungsplan abgerastert. Sofern es sich um ein bewegtes Zielvolumen handelt, wird der Bestrahlungsplan noch mit der Bewegung des Zielvolumens überlagert, so dass die dreidimensionale Positionsänderung des Zielvolumens, insbesondere in Echtzeit, kompensiert und gleichzeitig das Zielvolumen abgerastert werden kann.

Der Sensor erfasst während der Bestrahlung die Position des Zielvolumens fortlaufend, vorzugsweise kontinuierlich. Die Informationen, welche die Position des Zielvolumens beinhalten, werden der Regeleinrichtung zur Verfügung gestellt. Die Regeleinrichtung regelt bei einer Bewegung des Zielvolumens und somit in Ansprechen auf eine Positionsänderung des Zielvolumens die Strahlachse des Ionenstrahls nach, so dass die Positionsänderung des Zielvolumens kompensiert wird.

Die Regeleinrichtung erzeugt in Ansprechen auf eine Veränderung der Position des zielvolumens durch Bewegung ein Steuersignal, so dass die Ablenkung des Ionenstrahls in Ansprechen auf das Steuersignal derart angepasst wird, dass der Ionenstrahl einen entsprechenden Bereich der Reichweitenanpassungseinrichtung durchläuft, so dass die Positionsänderung des Zielvolumens durch die angepasste Reichweite des Ionenstrahls kompensiert wird und das Bragg-Maximum in den Bereich des zielvolumens geschoben wird.

Hierzu umfasst die Regeleinrichtung Mittel, welche in einem ersten Schritt die Positionen des Zielvolumens in Bezug zur Reichweite des Ionenstrahls setzen. Weiterhin umfasst sie Mittel, welche in einem nächsten Schritt die Reichweite des Ionenstrahls zu einem Auftreffort auf der Reichweitenanpassungseinrichtung in Beziehung setzen. Ferner umfasst sie Mittel, welche den Auftreffort auf der Reichweitenanpassungseinrichtung zu einer bestimmten Ablenkung durch die erste Ablenkeinrichtung setzen.

Das Steuersignal oder die Steuersignale, welches oder welche die erste Ablenkeinrichtung erhält bzw. welche die erste und die zweite Ablenkeinrichtung erhalten, enthält bzw. enthalten die Information über die Größe der Ablenkung des Ionenstrahls. Das Steuersignal ist oder die Steuersignale sind somit korreliert zu der Position des Zielvolumens.

Weiterhin ist die vorliegende Erfindung dadurch gekennzeichnet, dass die Regeleinrichtung die Reichweitenanpassung in Echtzeit, d.h. an eine Bewegung des Zielvolumens angepasst, durchführen kann oder dass die Reichweitenanpassung in Echtzeit durchführbar ist. Beispielsweise wird die Anpassung zyklisch, insbesondere periodisch, durchgeführt.

Die Regeleinrichtung kann mit einer Regelfrequenz in einem Bereich von größer als etwa 10 Hz, bevorzugt von größer als etwa 100 Hz, besonders bevorzugt von größer als etwa 1 kHz betrieben werden oder ist mit einer solchen Regelfrequenz betreibbar.

Weiterhin betrifft die vorliegende Erfindung eine Anlage zur therapeutischen Bestrahlung mit Ionenstrahlen, weiche einen Beschleuniger zum Erzeugen des Ionenstrahls und die vorstehend beschriebene Vorrichtung zum Anpassen der Reichweite des Ionenstrahls umfasst.

Die vorliegende Erfindung wird anhand der nachfolgenden Ausführungsbeispiele im Einzelnen erläutert. Hierzu wird auf die beigefügten Zeichnungen Bezug genommen. Die gleichen Bezugszeichen in den einzelnen Zeichnungen beziehen sich auf die gleichen Elemente.

### Figurenbeschreibung

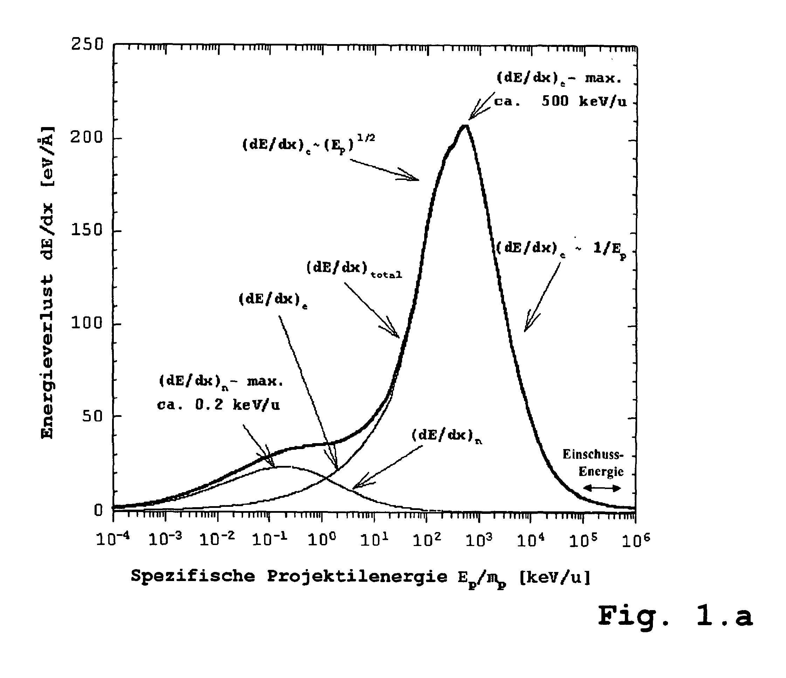

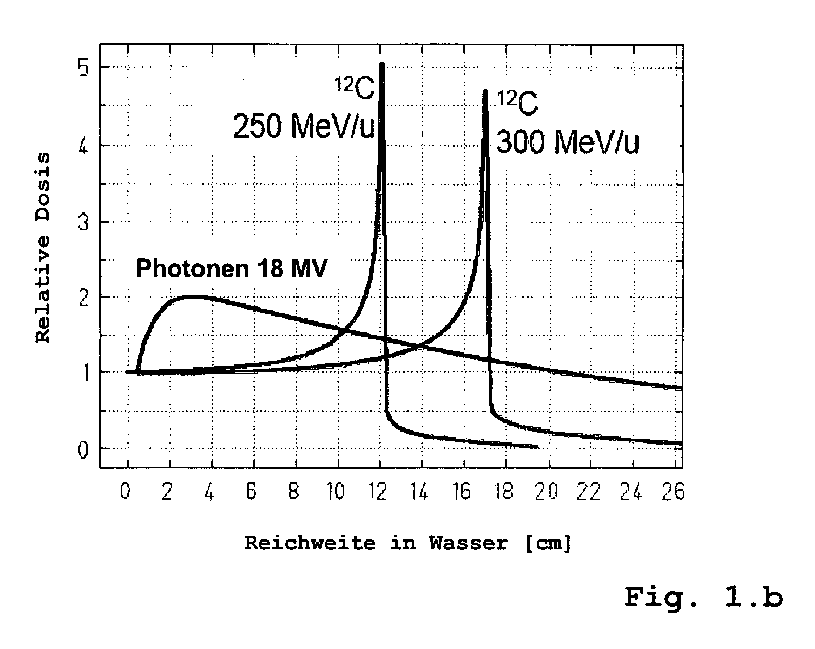

Fig. 1.a zeigt einen beispielhaften Verlauf des Energieverlustes eines Ions in einem Festkörper als Funktion der spezifischen Projektilenergie.
Fig. 1.b zeigt einen beispielhaften Verlauf der von einem Ionenstrahl in Wasser deponierten Dosis als Funktion der Eindringtiefe.
Fig. 2.a illustriert das Prinzip der lateralen und longitudinalen Anpassung eines Ionenstrahls an die Position des zu bestrahlenden Gewebes.

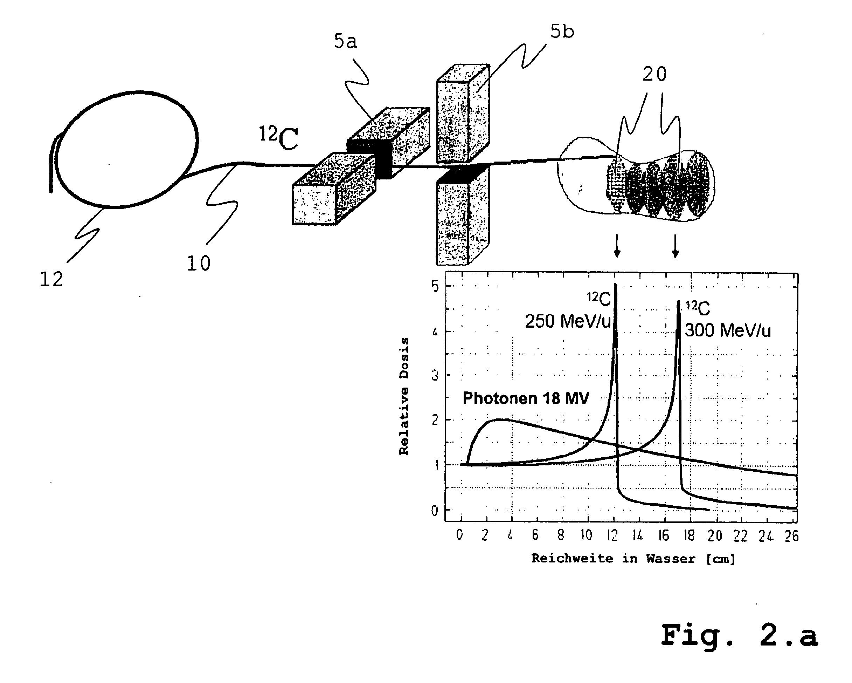

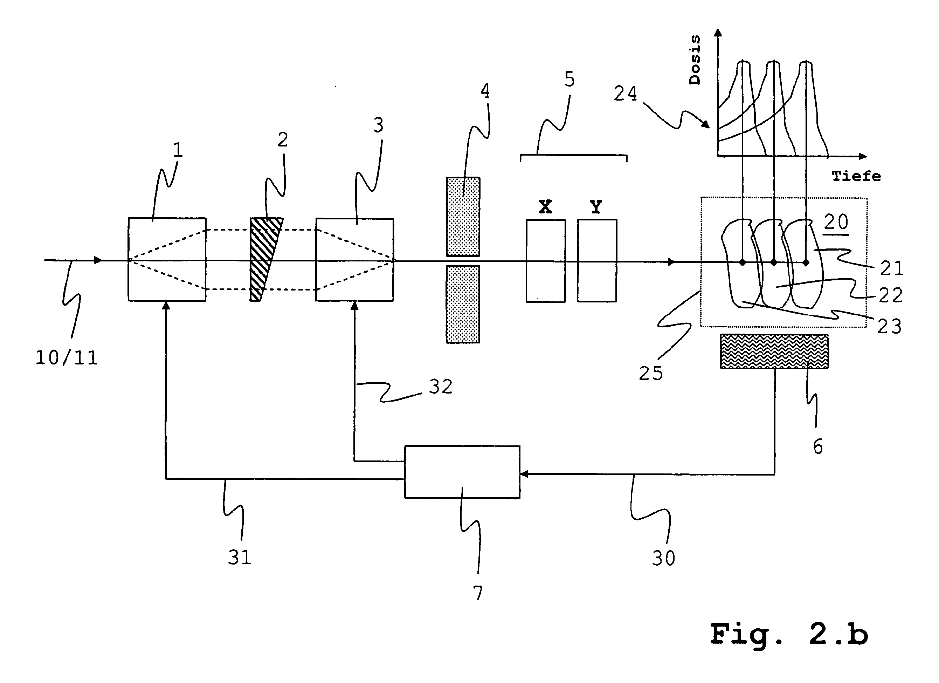

Fig. 2.b zeigt eine schematische Darstellung einer Vorrichtung, die das verständnis der Erfindung erleichtert.

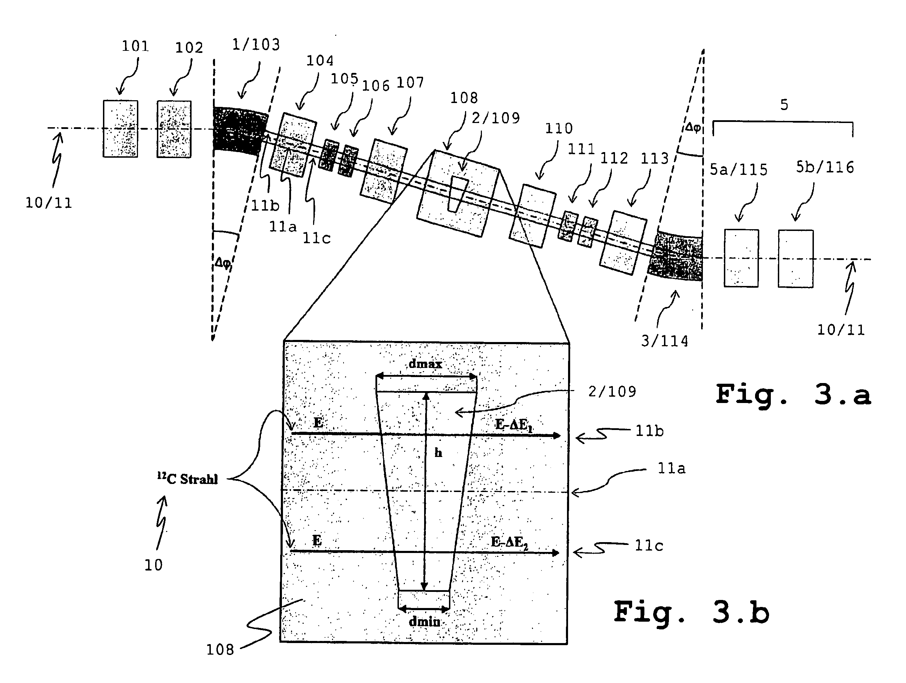

Fig. 3.a zeigt eine Detailansicht einer beispielhaften Strahlführung mit einer beispielhaften Ausführungsform der erfindungsgemäßen Vorrichtung.
Fig. 3.b zeigt eine Detailansicht der beispielhaft in Figur 3.a dargestellten Reichweitenanpassungseinrichtung.

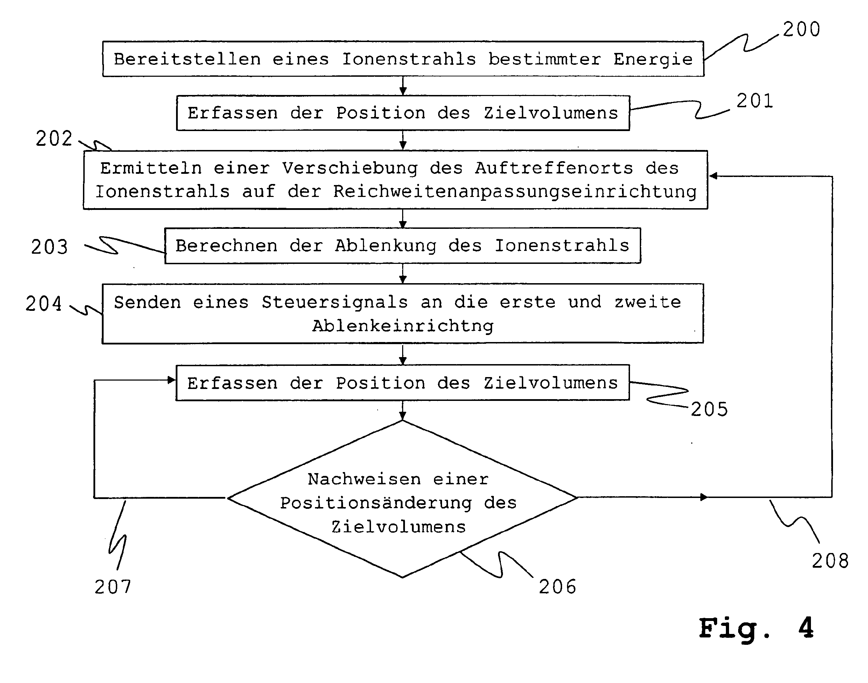

Fig. 4 zeigt schematisch in einem Ablaufdiagramm den Regelkreis zum Anpassen der Reichweite eines Ionenstrahls.

### Detaillierte Beschreibung der Erfindung

Krebspatienten mit lokalisierten Tumoren werden mit einem hochenergetischen Ionenstrahl 10, vorzugsweise mit einem Ionenstrahl 10 der Kohlenstoffionen umfasst, in einem Energiebereich von 10 MeV/u bis 600 MeV/u bestrahlt, entsprechend einer maximalen Eindringtiefe von etwa 40 cm Wasseräquivalent. Der Ionenstrahl 10 wird beim Durchlaufen des Körpergewebes 25 durch quasi-kontinuierliche Stoßprozesse mit den Atomen und Elektronen des Körpergewebe 25 abgebremst und verliert einen Teil seiner kinetischen Energie, welche in dem Körpergewebe 25 deponiert wird. Der mittlere Energieverlust pro Längeneinheit, bzw. die entlang der Strecke x deponierte Energie E, wird dabei durch den Energieverlust dE/dx ausgedrückt.

Figur 1.a zeigt dazu schematisch den beispielhaften Energieverlust dE/dx eines Ions aus einem Ionenstrahl 10 in einem Festkörper, wie zum Beispiel menschlichem Gewebe 25, als Funktion der spezifischen kinetischen Energie Eₚ/mₚ des Ions. Hierbei beschreibt Eₚ die kinetische Energie des Ions und mₚ die Masse des Ions. Der gesamte oder totale Energieverlust setzt sich aus dem nuklearen Energieverlust dE/dxₙ und dem elektronischen Energieverlust dE/dxₑ zusammen. Bei kleinen Projektil- oder Ionengeschwindigkeiten Eₚ wird der totale Energieverlust durch den nuklearen Energieverlust, mit einem Maximum bei etwa 0,2 keV/u, dominiert. Dagegen wird der totale Energieverlust bei hohen Ionengeschwindigkeiten Eₚ durch den elektronischen Energieverlust dominiert, mit einem Maximum bei etwa 0,5 MeV/u, dem sogenannten Bragg-Peak oder Bragg-Maximum.

Die durch das Abbremsen entlang der Ionenstrahl- oder Projektiltrajektorie im Gewebe 25 deponierte kinetische Energie führt zu einer Schädigung des Gewebes 25. Je höher die deponierte Energie pro Strecke bzw. pro Volumen im Gewebe 25 ist, desto höher ist auch dessen Schädigung. Im Bereich des Bragg-Peaks ist der Energieverlust und somit auch die Schädigung des Gewebes 25 am größten.

Ziel der Ionenstrahltherapie ist es, die maximale Schädigung im Zielvolumen 20, d.h. im Tumor, bei gleichzeitiger Schonung des umliegenden gesunden Gewebes zu erreichen. Dies kann über die entsprechende Wahl der Reichweite eines Ionenstrahls 10, welche mit der kinetischen Energie des Ionenstrahls 10 in direkten Zusammenhang steht, eingestellt werden. Das Prinzip der Ionenstrahltherapie wird dazu nachfolgend anhand von Figur 1.a am Beispiel eines statischen Tumors skizziert.

Der Ionenstrahl 10 wird mit einer definierten kinetischen Einschussenergie bereitgestellt. Hierbei wird der Ionenstrahl 10 beispielsweise mit einer spezifischen kinetischen Energie Eₚ/mₚ in einem Bereich von 1 bis 5 mal 10⁵ keV/u (100 bis 500 MeV/u) bereitgestellt. Diese liegt oberhalb des Bragg-Maximums und berücksichtigt unter anderem die Lage bzw. Tiefe des Tumors 20 im Gewebe 25. Dieser Energiebereich ist in Figur 1.a mit einem waagrechten Pfeil gekennzeichnet. Mit dieser Einschussenergie trifft der Ionenstrahl 10 auf die Oberfläche des Gewebes 25, was einer Eindringtiefe von 0 cm entspricht, auf. Der Ionenstrahl 10 trifft mit dieser Energie auf das Gewebe 25 und dringt in die ersten Lagen des Gewebes 25 ein. Die deponierte Energie pro Strecke dE/dx mit einem Wert von etwa 5 bis 10 eV/Å und die damit verbundene Schädigung des Gewebes 25 weist zunächst einen niedrigen Wert auf, insbesondere im Vergleich zum Bragg-Peak einen um einen Faktor von etwa 20 bis 40 niedrigeren Wert.

Beim Eindringen in das Gewebe 25 wird der Ionenstrahl 10 aufgrund der Wechselwirkung mit dem Gewebe 25 sukzessive abgebremst. Dadurch nimmt die kinetische Energie des Ionenstrahls 10 ab und der Energieverlust pro Strecke dE/dx steigt an. Bei einer Energie von Eₚ/mₚ gleich 10⁴ keV/u liegt der Energieverlust dE/dx bei etwa 50 eV/Å, bei einem niedrigeren Eₚ/mₚ von 10³ keV/u liegt der Energieverlust bei einem höheren Wert von etwa 185 eV/Å (rechte Flanke des Bragg-Peaks). Bei entsprechend der Lage des Zielvolumens angepasster kinetischer Energie des Ionenstrahls 10 wird der Ionenstrahl 10 derart abgebremst, dass er die erforderliche Energie Eₚ/mₚ von etwa 500 keV/u und somit das Bragg-Maximum mit einem beispielhaft dargestellten dE/dx von etwa 210 eV/Å im Zielvolumen 20 erreicht. Bei der Ionenstrahltherapie ist die kinetische Energie des Ionenstrahls 10 so gewählt, dass erreicht wird, dass der Bragg-Peak im Bereich des Zielvolumens 20 und somit im Bereich des Tumors 20 liegt. Da die Lage des Tumors 20 in unterschiedlichen Tiefen, ausgehend von dem Eindringort des Ionenstrahls befindlich ist, muss die kinetische Anfangsenergie des Ionenstahls dieser Tiefenlage entsprechend gewählt werden. Dies ist notwendig, damit die maximale Schädigung im Bereich des Tumors 20 erzielt wird. Da die kinetische Energie des Ionenstrahls 10 im Bereich des Tumors 20 weiter abnimmt, ist die im Gewebe 25 deponierte Energie sehr gering, wie aus der niederenergetischen Seite des Bragg-Peaks (linke Flanke) in Fig. 1.a deutlich erkennbar ist. Dadurch wird auch nur eine geringe oder sogar, beim vollständigen Abstoppen des Ionenstrahls 10 im Gewebe 25, keine Energie in dem Gewebe 25 hinter dem Zielvolumen 20 deponiert. Somit kann bei einer an die Tiefenlage des Tumors 20 angepassten kinetischen Energie des Ionenstrahls 10 gleichzeitig bei maximaler Schädigung eines Tumors 20 das umliegende Gewebe 25, d.h. sowohl das vor als auch hinter dem Tumor 20 liegende Gewebe 25, geschont werden.

Das der Ionenstahltherapie zugrunde liegende physikalische Phänomen ist bereits vorstehend zu Figur 1.a skizziert. Die Ionenstrahltherapie in ihrer tatsächlichen "Anwendung" wird noch einmal ergänzend anhand von Figur 1.b illustriert. Dargestellt ist hierbei nicht mehr die in einem Gewebe 25 deponierte Energie eines einzelnen Ions als Funktion der spezifischen kinetischen Energie wie in Figur 1.a. Sondern es ist die Dosis, d.h. die von einem Ionenstrahl 10 und somit von einer Vielzahl von Ionen in einem Körper 25 deponierten Energie, dargestellt. Die Dosis ist vorliegend als Funktion der Reichweite, welche identisch mit der Eindringtiefe ist, dargestellt und nicht, wie in Figur 1.a, als Funktion der spezifischen kinetischen Energie.

Die spezifische kinetische Energie korreliert jedoch mit der Eindringtiefe. In vereinfachten Worten: Figur 1.b beschreibt die Dosis, welche sich aus der Summe der von einzelnen Ionen in einem Körper deponierten Energie ergibt. Die relative Dosis ist eine auf einen Maximalwert im Bragg-Peak normierte Dosis. Dabei ist jedoch die spezifische kinetische Energie der Ionen, welche diese aufgrund der sukzessiven Abbremsung im Körper in verschiedenen Eindringtiefen im Körper besitzen, auf diese Eindringtiefe umgerechnet. Beispielsweise entspricht eine Eindringtiefe von 0 cm dem in Figur 1.a markierten Bereich der Einschussenergie. Beim Eindringen in das Gewebe 25, d.h. mit zunehmender Eindringtiefe, steigt die im Gewebe deponierte Dosis an, bis diese das Maximum, den Bragg-Peak, erreicht, und fällt dann wieder ab.

Der in Figur 1.b dargestellte Verlauf der relativen Dosis als Funktion der Eindringtiefe ergibt sich aus dem in Figur 1.a illustrierten Phänomen, dass die von einem Ion in einem Körper deponierte Energie abhängig ist von dessen spezifischer kinetischer Energie. Diese ist wiederum abhängig von der Eindringtiefe des Ions im Körper abhängig, da das Ion im Körper sukzessive abgebremst wird.

Figur 1.b zeigt hierzu einen beispielhaften Verlauf der von einem Ionenstrahl 10 in einem Körper 25, hier am Beispiel von Wasser als ein wesentlicher Bestandteil eines Gewebes, deponierten Dosis als Funktion der Tiefe im Körper 25. Die Tiefendosisverteilung ist für ein ¹²C-Ionenstrahl mit einer spezifischen Energie von 250 MeV/u und 300 MeV/u vergleichend dargestellt. Diese Energien liegen in dem in Figur 1.a markierten Bereich der Einschussenergien (waagrechter Pfeil). Zum Vergleich und zum Beleg der Vorteile von Ionenstrahlen 10 gegenüber Photonenstrahlung ist zusätzlich die Tiefendosisverteilung für Photonen der Energie 18 MV dargestellt. Das Dosismaximum für die Photonen befindet sich kurz unterhalb der Oberfläche des Körpers 25. Dagegen liegt das jeweilige Dosismaximum für den Ionenstrahl 10 in der Tiefe des Körpers 25 oder des Gewebes. Die Photonendosis nimmt exponentiell ab. Dagegen besitzt die Ionendosis ein relativ scharfes Maximum, den Bragg-Peak, und einen relativ scharfen Abfall oder "Cut-Off" nach dem Maximum. Somit kann die maximale Dosis, d.h. die maximale Schädigung, insbesondere scharf definiert bzw. lokalisiert in der Tiefe des Gewebes eingebracht werden. Zudem kann durch eine Veränderung der spezifischen Energie des Ionenstrahls 10 die Position des Bragg-Peaks in der Tiefe verändert bzw. angepasst werden. Die Breite des Bragg-Maximums bzw. des Bragg-Peaks resultiert aus der statistischen Natur eines Mehrfachstreuprozesses. Es ergibt sich hierbei eine annähernd gaußförmige Reichweitenverteilung. Der beobachtete Bragg-Peak ergibt sich aus der Summe der Tiefendosisverteilung einer Vielzahl einzelner Teilchen bzw. Ionen. Die Faltung einer scharfen Einzelteilchendosis mit einer gaußförmigen Reichweitenverteilung führt zu der Breite des Bragg-Peaks.

Die vorliegende Erfindung befasst sich mit der Anpassung der Eindringtiefe oder Reichweite des Ionenstrahls 10, d.h. der Position des Bragg-Maximums in einem zu bestrahlenden bewegten Gewebe 25. Während, in Strahlrichtung gesehen, seitliche Verschiebungen des zielvolumens 20 mittels einer schnellen Ansteuerung der Rasterscan-Magnete 5 kompensiert werden können, erfordern Verschiebungen in Strahlrichtung eine schnelle Anpassung der spezifischen Energie der Ionen 10 und damit der Position des Bragg-Maximums in der Tiefe des Gewebes 25. Figur 2.a illustriert hierzu das Prinzip der lateralen und longitudinalen Anpassung des Ionenstrahls 10 bzw. des Bragg-Peaks an die Position des zu bestrahlenden Gewebes oder Zielvolumens 20. Die eingefügte Grafik entspricht der in Figur 1.b gezeigten Graphik. Der Ionenstrahls 10 wird von einem Beschleuniger 12, hier einem Synchrotron, mit einer bestimmten spezifischen Energie bereitgestellt. Über eine Anpassung der spezifischen Energie wird die longitudinale Lage des Bragg-Peaks eingestellt, insbesondere zum Anpassen an die Position oder Tiefe des Zielvolumens 20 im Gewebe und/oder zum longitudinalen Scannen des Zielvolumens 20. Mittels der Dipol-Magnete 5a und 5b oder der Rasterscan-Magnete 5a und 5b wird die transversale Lage des Ionenstrahls 10 bzw. des Bragg-Peaks eingestellt, insbesondere zum Anpassen an die transversale Position des Zielvolumens 20 und/oder zum transversalen Scannen des Zielvolumens 20. Mittels der Rasterscan-Magnete 5a und 5b erfolgt eine Ablenkung in horizontaler bzw. vertikaler Ebene.

Figur 2.b zeigt eine schematische Darstellung einer Vorrichtung, die das Verständnis der Erfindung erleichtert. Es wird ein Keil als Reichweitenmodulator 2 oder ein einfacher keil- oder stufenförmiger Reichweitenmodulator 2 eingesetzt, der sich innerhalb der Strahlführung vor den Rasterscan-Magneten 5 befindet. Der Reichweitenmodulator 2 kann gemäß seiner Funktion auch als Energieverlust-Keil bezeichnet werden. Vorzugsweise wird der Reichweitenmodulator 2 nicht bewegt. Er ist örtlich fest und somit statisch eingebaut. Im Stand der Technik wird die Anpassung der Reichweite mittels zweier gegeneinander verschiebbarer keilförmiger Reichweitenmodulatoren erreicht. Der Ionenstrahl wird hierbei nicht bewegt. Sondern es werden die beiden Reichweitenmodulatoren zueinander bewegt, was jedoch aufgrund der notwendigen Präzision und der großen Massen der Modulatoren sehr aufwendig ist.

Der Ionenstrahl 10 wird durch einen nicht dargestellten Beschleuniger bereitgestellt. Beispielhaft sei ein Synchrotron-Beschleuniger genannt. Die Einstellung der Reichweite oder die Energie- bzw. Reichweitenmodulation wird dadurch erreicht, dass der Ionenstrahl 10 von einer ersten Ablenkeinrichtung 1, einer schnellen magnetischen Ablenkeinheit, in einer Ebene senkrecht zum Ionenstrahl 10 aus der Strahlachse 11 heraus so abgelenkt wird, dass er den Reichweitenmodulator 2 an Stellen unterschiedlicher Dicke durchdringt. Es ist ein Reichweitenmodulator 2, in welchem der Ionenstrahl 10 beim Durchqueren einen Energieverlust erfährt, wobei der Reichweitenmodulator 2 an unterschiedlichen Stellen einen unterschiedlichen Energieverlust bewirkt, um die Reichweite des Ionenstrahls 10 im Patientengewebe gezielt einzustellen. Der Reichweitenmodulator 2, welcher auch als Reichweitenanpassungseinrichtung 2 bezeichnet wird, ist vorliegend beispielhaft keilförmig ausgebildet. Er besitzt eine unterschiedliche Dicke in der Ebene, in welcher der Ionenstrahl 10 abgelenkt wird.

In Strahlrichtung hinter dem Reichweitenmodulator 2 wird der Ionenstrahl 10 mittels einer zweiten Ablenkeinrichtung 3, einer weiteren schnellen magnetischen Ablenkeinheit, auf die ursprüngliche zentrale Strahlachse 11 zurückgelenkt, sodass der Ionenstrahl 10 unabhängig von der jeweiligen Ionengeschwindigkeit den vorgegebenen Zielpunkt im Zielvolumen 20 erreicht. Vorliegend wird der Ionenstrahl 10 nach der Reichweitenanpassung somit auf die ursprüngliche Strahlachse 11 zurück und in Richtung auf das Zielvolumen 20 gelenkt. Im Detail wird der Ionenstrahl 10 somit vor dem Passieren des Reichweitenmodulators 2 in einem ersten Schritt aus seiner Strahlachse 11 abgelenkt. In einem zweiten Schritt wird er auf eine Achse gelenkt, die seitlich versetzt, insbesondere parallel und seitlich versetzt, zur Strahlachse 11 verläuft. In einem dritten Schritt wird der Ionenstrahl aus dieser seitlich versetzten Achse in Richtung der ursprünglichen Achse 11 abgelenkt. In einem vierten Schritt wird der Ionenstrahl dann wieder auf die ursprüngliche Achse 11 zurückgelenkt.

Der in Strahlrichtung hinter der zweiten Ablenkeinrichtung 3 positionierte Kollimator 4 dient dazu, die durch Winkelstreuung im Reichweitenmodulator 2 bewirkte Strahlaufweitung zu begrenzen. In Kombination mit der zweiten Ablenkeinrichtung 3 stellt er zudem einen Impulsfilter dar, der nur Ionen der gewünschten Geschwindigkeit bzw. kinetischen Energie passieren lässt. Aus Sicherheitsaspekten ermöglicht dies eine präzise Filterung der gewünschten kinetischen Energie. In Strahlrichtung nach dem Kollimator 4 befinden sich die vorstehend bereits erwähnten Rasterscan-Magnete 5, wobei zwei Magnete umfasst sind und der Ionenstrahl 10 in einer XY-Ebene senkrecht zur Strahlachse 11 über das Zielvolumen 20 bei entsprechender lateraler Ausdehnung geführt werden kann.

Der Körper oder das Gewebe 25, in dem sich das Zielvolumen 20 befindet, ist schematisch durch die gestrichelten Linien dargestellt. Im Bereich des Zielvolumens 20 ist ein Sensor 6, vorzugsweise ein Bewegungssensor 6, angeordnet, welcher die Position und/oder eine Bewegung des Zielvolumens 20 erfasst. Der Bewegungssensor 6 entspricht dem Stand der Technik. Beispielhaft seien hier ein Anzai-Gürtel, ein Video-System oder im Gewebe implantierte Sonden genannt.

Durch eine, zum Beispiel atmungsbedingte, Bewegung der Lungen oder der Weichteile eines Patienten kann sich die Position des Zielvolumens 20 sowohl entlang der Strahlachse als auch seitlich zur Strahlachse 11 verändern, so dass der Bragg-Peak aus dem Zielvolumen 20 herausgeschoben werden kann und sich der Ort der maximalen Schädigung nunmehr unter Umständen im gesunden Gewebe des Körpers 25 und nicht mehr im Zentrum des Tumors 20 befindet. Dies ist anhand der drei Positionen 21, 22 und 23 des Zielvolumens 20 illustriert. Dies erfordert sowohl eine schnelle Korrektur der lateralen Position des Ionenstrahls 10 als auch eine schnelle Anpassung der kinetischen Energie des Ionenstrahls 10 und damit der Eindringtiefe.

Seitliche Verschiebungen des Zielvolumens 20, relativ zur Strahlachse 11, können mittels einer nicht dargestellten schnellen Ansteuerung der Rasterscan-Magnete 5 kompensiert werden. Verschiebungen in Strahlrichtung erfordern jedoch eine Kompensation durch eine schnelle Anpassung der spezifischen Energie der Ionen 10 und damit der Position des Bragg-Maximums in der Tiefe des Gewebes 25.

Um eine schnelle Anpassung der Reichweite des Ionenstrahls, die auch als Ionenreichweite bezeichnet wird, zu erreichen, wird die Bewegung des Zielvolumens 20 von dem Bewegungssensor 6, insbesondere zeitlich aufgelöst, erfasst. Es wird eine Information 30 über die Position des Zielvolumens 20, wie ein Messwert, an eine Regeleinrichtung 7 geschickt. Aus der Änderung der Messwerte bzw. der Positionsänderung berechnet die Regeleinrichtung 7 die erforderliche Änderung der Ionenreichweite und schickt ein entsprechendes Korrektursignal 31 an die erste Ablenkeinrichtung 1 und ein entsprechendes Korrektursignal 32 an die zweite Ablenkeinrichtung 3.

Das Korrektur- oder Steuersignal 31 ist zur aktuellen Position bzw. Tiefenlage des Zielvolumens 20 derart korreliert, dass der Ionenstrahl 10 aus der Strahlachse 11 heraus auf den entsprechenden Bereich des Reichweitenmodulators 2 gelenkt wird, so dass der Energieverlust in dem Reichweitenmodulator 2 an die Tiefenlage des Zielvolumens 20 angepasst ist. Das Korrektur- oder Steuersignal 32 ist zur aktuellen Position bzw. Tiefenlage des Zielvolumens 20 derart korreliert, dass der Anteil des Ionenstrahls 10, welcher nach dem Energieverlust in dem Reichweitenmodulator 2 die erforderliche Reichweite besitzt, wieder auf die ursprüngliche Strahlachse 11 zurückgelenkt wird.

Mittels des Reichweitenmodulators 2 ist nur eine Verringerung der Reichweite des Ionenstrahls 10 zu erzielen. Folglich ist die Energie des Ionenstrahls 10, welche vom Beschleuniger bereitgestellt wird, auf die maximale Tiefe des Zielvolumens 20 abzustimmen. D.h. der Ionenstrahl 10 wird vor dem Eintritt in den Reichweitenmodulator 2 mit einer kinetischen Energie bereitgestellt, die ausreichend groß ist, um die maximale Tiefe des Zielvolumens 20, hier die Position 21, erreichen oder auch noch tiefer in den Körper 25 eindringen zu können. Weniger tief liegende Positionen, hier die Positionen 22 und 23 des Zielvolumens 20, können durch eine Energie- bzw. Tiefenanpassung mittels des Reichweitenmodulators 2 erreicht werden. Die Verschiebung des Bragg-Peaks in dem Zielvolumen 20 ist mittels der Graphik 24, welche den Energieverlust bzw. die Dosis in Abhängigkeit von der Tiefe bzw. Eindringtiefe zeigt, illustriert. Die maximale Dosis ist jeweils in Echtzeit an die aktuelle Position 21, 22, 23 des Zielvolumens 20, d.h. an eine Bewegung des Zielvolumens 20, angepasst. Die erfindungsgemäße Reichweitenanpassung kann auch bei ausgedehnten statischen Zielvolumen 20 angewendet werden, um das Zielvolumen 20 in verschiedenen Tiefen lagenweise zu bestrahlen.

Ein Vorteil liegt darin begründet, dass die Variation der Dicke des Reichweitenmodulators 2 nicht mechanisch sondern durch Einstellung von Magnetfeldern bzw. Magnetatrömen erfolgt. Damit wird eine Reichweitenanpassung mit wesentlich höherer Geschwindigkeit bzw. Regelfrequenz ermöglicht als mittels mechanischer verschiebung.

Zudem befindet sich der Reichweitenmodulator 2 im Unterschied zum Stand der Technik nicht unmittelbar vor dem Patienten bzw. Zielvolumen 20. Er befindet sich stattdessen strahlaufwärts in der Strahlführung gesehen vor den Rasterscan-Magneten 5. Da der Ionenstrahl 10 vor der ersten Ablenkeinrichtung 1 gut fokussiert bereitgestellt werden kann, z.B. mit einem Strahldurchmesser von etwa 5 mm, können die Abmessungen des Reichweitenmodulators 2 klein gehalten werden. In einer Ausführungsform ist der Reichweitenmodulator 2 etwa 6 cm lang und 1 cm breit. Demgegenüber muss ein direkt vor dem Patienten angeordnetes Keilsystem zur Modulation gemäß dem Stand der Technik die volle Größe des Bestrahlungsfeldes von etwa 20 x 20 cm² abdecken. Dies wiederum führt zu beträchtlichen Massen bzw. Gewichten der Keile und entsprechend hohen Anforderungen an die Linearachsenantriebe. Weiterhin führt die schnelle Bewegung der bekannten Keilantriebe zu erheblichen Fahrgeräuschen, die für Patienten unangenehm werden können. Die genannten Nachteile werden durch die Erfindung vermieden.

Weiterhin können bei der Anordnung im Stand der Technik sekundäre Fragmente, insbesondere Neutronen, die infolge von Kernreaktionen in einem Reichweitenmodulator 2 erzeugt werden, nicht mehr vom Ionenstrahl 10 getrennt werden, wenn sich der Reichweitenmodulator 2, wie im Stand der Technik, unmittelbar vor dem Patienten befindet. Wegen der stark vorwärtsgerichteten Winkelverteilung der Fragmente laufen diese dann direkt in den Patienten und erzeugen eine unkontrollierte, unerwünschte und zusätzliche Dosis.

Figur 3.a zeigt eine Detailansicht einer beispielhaften Führung des Ionenstrahls 10 mit einer Ausführungsform der vorliegenden Erfindung. Der Ionenstrahl 10 wird bereitgestellt und tritt entlang der Achse 11 in die Strahlführung ein. Insbesondere zur Führung und Fokussierung des Ionenstrahls 10 sind in Strahlrichtung vor dem Energieverlust-Keil 2 die Quadrupolmagnete 101 und 102 angeordnet. Mittels der ersten Ablenkeinrichtung 1, vorliegend ausgebildet als ein horizontaler Dipol 103, ist der Ionenstrahl 10 aus seiner ursprünglichen Achse 11 heraus und auf eine Achse 11a ablenkbar, welche zur ursprünglichen Achse 11 geneigt ist. Auf dieser Achse 11a ist der Energieverlust-Keil 2 angeordnet. In Abhängigkeit von der angelegten Stärke des vorzugsweise magnetischen Feldes der ersten Ablenkeinrichtung 1, insbesondere bei konstanter spezifischer Energie des Ionenstrahls 10, ist der Ionenstrahl 10 auf unterschiedliche Achsen 11a, 11b, 11c lenkbar und hierbei insbesondere auch auf unterschiedliche Orte auf dem Energieverlust-Keil 2 lenkbar. Vorliegend wird der Ionenstrahl 10 hierbei auf die Achsen 11a, 11b, 11c gelenkt, welche im Wesentlichen parallel und seitlich versetzt zueinander verlaufen.

Vor dem Auftreffen auf dem Energieverlust-Keil 2 durchläuft der Ionenstrahl 10 vorliegend, insbesondere zur Strahlführung und/oder -formung, einen Quadrupolmagneten 104, einen insbesondere horizontalen Steeringmagneten 105, einen insbesondere vertikalen Steeringmagneten 106 und einen weiteren Quadrupolmagneten 107. Vorzugsweise sind die vier Komponenten 104, 105, 106 und 107 in Strahlrichtung in dieser Reihenfolge angeordnet.

Der Ionenstrahl 10 trifft auf den Energieverlust-Keil 2 auf. In Abhängigkeit von seiner Achse 11a, 11b, 11c trifft der Ionenstrahl 10 auf unterschiedlichen Orten auf dem Energieverlust-Keil 2 auf und durchläuft diesen in unterschiedlichen Bereichen. Der Energieverlust-Keil 2 besitzt in einer Ausdehnung senkrecht zur Strahlachse 11a, hier der Breite h, eine unterschiedliche Dicke. Somit erfährt der Ionenstrahl 10 in Abhängigkeit von seinem Auftreffort auf dem Energieverlust-Keil 2 einen unterschiedlichen Energieverlust bzw. einen definierten Energieverlust. Der Energieverlust-Keil 2 ist beispielhaft in einer Vakuumkammer 108 angeordnet. Der Energieverlust-Keil 2 ist vorliegend als ein trapezförmiger Energieverlust-Keil 109 ausgebildet. Er wird nachfolgend in der Figur 3.b detailliert beschrieben.

Nach dem Passieren des Energieverlust-Keils 2 durchläuft der Ionenstrahl 10, insbesondere zur Strahlführung und/oder -formung, einen Quadrupolmagneten 110, einen insbesondere horizontalen Steeringmagneten 111, einen insbesondere vertikalen Steeringmagneten 112 und einen weiteren Quadrupolmagneten 113. Vorzugsweise sind die vier Komponenten 110, 111, 112 und 113 in Strahlrichtung in dieser Reihenfolge angeordnet. Die vier Komponenten 104, 105, 106 und 107 und die vier Komponenten 110, 111, 112 und 113 können auch in ihrer Anordnung im Wesentlichen spiegelsymmetrisch zum Energieverlust-Keil 2 angeordnet sein.

Mittels der zweiten Ablenkeinrichtung 3, vorliegend ausgebildet als ein horizontaler Dipol 114, ist der Ionenstrahl 10 aus der Achse 11a, 11b, 11c heraus und auf eine Achse 11 ablenkbar, welche zur Achse 11a, 11b, 11c geneigt ist. Auf dieser Achse 11 sind vorliegend die Rasterscan-Magnete 5 bzw. 5a und 5b angeordnet. Mittels des vorzugsweise horizontalen Dipols 114 wird somit der Ionenstrahl 10 auf die Rasterscan-Magnete 5 bzw. 5a und 5b gelenkt. Die Rasterscan-Magnete 5a und 5b sind insbesondere als Quadrupolmagnete 115 und 116 ausgebildet. Vorzugsweise wird der Ionenstrahl 10 hierbei auf eine Achse 11 gelenkt, welche parallel und seitlich versetzt zur ursprünglichen Achse 11, auf welcher der Ionenstrahl 10 in die erste Ablenkeinrichtung 1 eintritt, verläuft.

Die einzelnen Komponenten der Strahlführung sind noch einmal zur Übersicht in Tabelle 1 zusammengefasst.

Figur 3.b zeigt eine Detailansicht des in Figur 3.a dargestellten Energieverlust-Keils 2. Der Energieverlust-Keil 2 ist beispielhaft ausgebildet in der Form eines, vorzugsweise gleichschenkligen, Trapezes 108. Der trapezförmige Energieverlust-Keil 108 besitzt die Höhe h und die Grundseiten dₘₐₓ und dₘᵢₙ. Die Höhe h beschreibt die Breite des Keils 2, vorzugsweise in der horizontalen Ebene. Die Achse bzw. die Höhe h des Energieverlust-Keils 2 oder des Trapezes steht senkrecht zur Strahlachse 11. Die Länge dₘᵢₙ beschreibt die minimale Dicke des Keils 2. Die Länge dₘₐₓ beschreibt die maximale Dicke des Keils 2. Weist die Länge dₘᵢₙ den Wert Null oder annähernd Null auf, geht das dargestellte Trapez 108 in ein, vorzugsweise gleichschenkliges, Dreieck über.

Der Ionenstrahl 10 wird als ein ¹²C-Strahl mit der Energie E bereitgestellt. Wird der Ionenstrahl 10 auf die dargestellte obere Achse 11b gelenkt, durchläuft der Ionenstrahl 10 den Energierverlust-Keil 2 somit an der oberen Stelle und erleidet einen Energieverlust ΔE1. Somit besitzt der Ionenstrahl 10 nach dem Passieren des Keils 2 die Gesamtenergie E-ΔE1. Wird der Ionenstrahl 10 dagegen auf die dargestellte untere Achse 11b gelenkt, durchläuft der Ionenstrahl 10 den Energierverlust-Keil 2 an der unteren Stelle und erleidet, auf Grund der geringeren Dicke des Keils 2 an dieser Stelle, einen geringeren Energieverlust ΔE2. Somit besitzt der Ionenstrahl 10 nach dem Passieren des Keils 2 die höhere Gesamtenergie E-ΔE2. Durchläuft der Ionenstrahl 10 den Energierverlust-Keil 2 an einer dazwischen liegenden Stelle, beispielsweise entlang der Achse 11a, erfährt dieser einen Energieverlust, welcher größer als ΔE2 und kleiner als ΔE1 ist. Somit lässt sich durch eine Variation des Auftreffortes auf dem Keil 2, der Energieverlust im Keil 2 und damit die resultierende Energie nach dem Keil 2 des Ionenstrahls 10 gezielt einstellen.

**Tabelle 1:**

| Bezugszeichen | Bezeichnung | Eigenschaften |
|---|---|---|
| 101 | Quadrupolmagnet | 1.Duplett, 1.Linse, 1 = 1000 mm, Δx = 60 mm, Δy = 60 mm |
| 102 | Quadrupolmagnet | 1.Duplett, 2.Linse, 1 = 1000 mm, Δx=60 mm, Δy = 60 mm |
| 103 | Horizontaler Dipol | Δϕ = -14.5°, ρ = 6.24998 m, 1 = 1582 mm, Δx=75 mm, Δy = 75 mm |
| 104 | Quadrupolmagnet | 2.Duplett, 1.Linse, 1 = 1000 mm, Δx =60 mm, Δy = 60 mm |
| 105 | Horizontaler Steeringmagnet | 1 = 200 mm, Δx =60 mm, Δy = 60 mm |
| 106 | Vertikaler Steeringmagnet | 1 = 200 mm, Δx=60 mm, Δy = 60 mm |
| 107 | Quadrupolmagnet | 2.Duplett, 2.Linse, 1 = 1000 mm, Δx=60 mm, Δy = 60 mm |
| 108 | Vakuumkammer mit Keil | |
| 109 | Keil (oder "Energy Degrader") | z.B. AlMg₃, ρ = 2.8 g/cm³, dₘᵢₙ = 0 mm, dₘₐₓ = 25 mm, h = 70 mm, ΔEₘₐₓ ≈ |
| | | 95 MeV/u |
| 110 | Quadrupolmagnet | 3.Duplett, 1.Linse, 1 = 1000 mm, Δx =60 mm, Δy = 60 mm |
| 111 | Horizontaler Steeringmagnet | 1 = 200 mm, Δx =60 mm, Δy = 60 mm |
| 112 | Vertikaler Steeringmagnet | 1 = 200 mm, Δx=60 mm, Δy = 60 mm |
| 113 | Quadrupolmagnet | 3.Duplett, 2.Linse, 1 = 1000 mm, Δx=60 mm, Δy = 60 mm |
| 114 | Horizontaler Dipol | Δϕ = -14.5°, ρ = 6.24998 m, 1 = 1582 mm, Δx=75 mm, Δy = 75 mm |
| 115 | Quadrupolmagnet | 4.Duplett, 1.Linse, 1 = 1000 mm, Δx=60 mm, Δy = 60 mm |
| 116 | Quadrupolmagnet | 4.Duplett, 2.Linse, 1 = 600 mm, Δx=60 mm, Δy = 60 mm |

Die in der Tabelle 1 verwendeten Parameter sind nachfolgend erläutert: Der Parameter 1 beschreibt die effektive Länge des Elementes, Δx die horizontale Apertur des Elementes (innerer Radius der Apertur), Δy die vertikale Apertur des Elementes (innerer Radius der Apertur), Δϕ den nominalen Ablenkwinkel, p den nominalen Radius des Dipols oder die Dichte des Keils, dₘᵢₙ die minimale Dicke des Keils, dₘₐₓ die maximale Dicke des Keils und h die Breite des Keils (horizontale Ebene).

Figur 4 zeigt in einem Flussdiagramm den Regelkreis zum Anpassen der Reichweite und der Lage des Bragg-Peak des Ionenstrahls 10 im Gewebe 25. Zunächst wird in einem ersten Schritt 200 ein Ionenstrahl 10 bestimmter Energie bereitgestellt. Die Energie ist an die Lage oder Tiefe des Zielvolumens 20 angepasst. Die Energie ist zumindest gleich der Energie oder größer als die Energie, die erforderlich ist, um die maximale Tiefe oder, bei Bewegung des Zielvolumens 20, die maximale Auslenkung des Zielvolumens 20, welches zum Beispiel der Position 21 in Figur 2.b entspricht, zu erreichen. In dem Schritt 201 wird die Position 21, 22, 23 des Zielvolumens 20 erfasst. In einem nächsten Schritt 202 wird die Verschiebung des Auftreffortes auf der Reichweitenanpassungseinrichtung 2 ermittelt. Trifft der Ionenstrahl 10 auf diesen Ort der Reichweitenanpassungseinrichtung 2 auf, erleidet er den entsprechenden Energieverlust beim Durchgang durch die Reichweitenanpassungseinrichtung 2, so dass die Reichweite des Ionenstrahls 10 an die Tiefe des Zielvolumens 20 angepasst ist. In einem nachfolgenden Schritt 203 wird die Ablenkung des Ionenstrahls 10 berechnet oder ermittelt, die erforderlich ist, damit der Ionenstrahl 10 auf den zuvor ermittelten Ort auf der Reichweitenanpassungseinrichtung 2 auftrifft. Ein der Ablenkung entsprechendes Steuersignal 31, 32 wird in dem Schritt 204 an die erste bzw. zweite Ablenkeinrichtung 1 bzw. 3 gesendet. Nach dem Passieren der ersten Ablenkeinrichtung 1, der Reichweitenanpassungseinrichtung 2 und der zweiten Ablenkeinrichtung 3 weist der Ionenstrahl 10 die an die Tiefe des Zielvolumens angepasste Reichweite auf. In einem nächsten Schritt 205 wird wieder die Position des Zielvolumens 20 erfasst. In einer Abfrage 206 wird festgestellt, ob eine Positionsänderung des Zielvolumens 20 vorliegt. Liegt keine Positionsänderung vor, wird in einer Prozessschleife oder Loop 207 der vorige Schritt 206 erreicht und erneut die Position des Zielvolumens 20 bestimmt oder erfasst. Liegt dagegen eine Positionsänderung vor, wird in einem Loop 208 der vorige Schritt 202 erreicht. Die Schritte 202 bis 208 werden erneut durchlaufen und die Reichweite des Ionenstrahls 10 wird in Echtzeit an die Bewegung 21, 22, 23 des Zielvolumens 20 innerhalb des Körpergewebes 25 angepasst. Die Schritte 202 bis 208 werden während der Bestrahlung fortlaufend, insbesondere kontinuierlich, durchgeführt.

Es ist dem Fachmann ersichtlich, dass die vorstehend beschriebenen Ausführungsformen beispielhaft zu verstehen sind. Die Erfindung ist nicht auf diese beschränkt, sondern kann in vielfältiger Weise variiert werden.

### Bezugszeichenliste

| | |
|---|---|
| 1 | Erste Ablenkeinrichtung |
| 2 | Reichweitenanpassungseinrichtung oder Reichweitenmodulator oder Energieverlust-Keil oder Keil |
| 3 | Zweite Ablenkeinrichtung |
| 4 | Kollimator |
| 5 | Rasterscan-Magnete |
| 5a | Rasterscan-Magnete zum Ablenken in horizontaler Ebene |
| 5b | Rasterscan-Magnete zum Ablenken in vertikaler Ebene |
| 6 | Sensor oder Bewegungssensor |
| 7 | Regeleinrichtung |
| 10 | Ionenstrahl |
| 11 | Strahlachse |
| 11a | Strahlachse |
| 11b | Strahlachse |
| 11c | Strahlachse |
| 12 | Beschleuniger |
| | |
| 20 | Zielvolumen oder Tumor |
| 21 | Erste Position des Zielvolumens |
| 22 | Zweite Position des Zielvolumens |
| 23 | Dritte Position des Zielvolumens |
| 24 | Graphik |
| 25 | Körper oder Körpergewebe |
| | |
| 30 | Positionsinformation |
| 31 | Steuersignal oder Korrektursignal für die erste Ablenkeinrichtung |
| 32 | Steuersignal oder Korrektursignal für die zweite Ablenkeinrichtung |
| | |
| 101 | Quadrupolmagnet |
| 102 | Quadrupolmagnet |
| 103 | Horizontaler Dipol |
| 104 | Quadrupolmagnet |
| 105 | Horizontaler Steeringmagnet |
| 106 | Vertikaler Steeringmagnet |
| 107 | Quadrupolmagnet |
| 108 | Vakuumkammer mit Keil |
| 109 | Keil (oder "Energy Degrader") |
| 110 | Quadrupolmagnet |
| 111 | Horizontaler Steeringmagnet |
| 112 | Vertikaler Steeringmagnet |
| 113 | Quadrupolmagnet |
| 114 | Horizontaler Dipol |
| 115 | Quadrupolmagnet |
| 116 | Quadrupolmagnet |
| 200 - 208 | Verfahrensschritte im Regelkreis |

## Patentansprüche

1. Vorrichtung zur Kompensation der Bewegung eines Zielvolumens (20) in Strahlrichtung bei der therapeutischen Behandlung eines Patienten mittels Bestrahlung des Zielvolumens (20) durch Ionen, indem die Reichweite des Ionenstrahls (10) an die Bewegung des zielvolumens (20) angepasst wird, umfassend
- eine Reichweitenanpassungseinrichtung (2,109) für den Ionenstrahl (10), in welcher der Ionenstrahl (10) beim Durchqueren unterschiedlicher Bereiche der Reichweitenanpassungseinrichtung (2,109) einen unterschiedlichen Energieverlust erfährt,
- einen Sensor (6) zur Erfassung der Position (21, 22, 23) des Zielvolumens (20), welcher eine die Position (21, 22, 23) beschreibende Information (30) bereitstellt,
- eine Regeleinrichtung (7), welche die Information (30) des Sensors (6) empfängt und ein zur Position (21, 22, 23) des Zielvolumens (20) korreliertes Steuersignal (31) erzeugt und
- eine steuerbare erste Ablenkeinrichtung (1,103), an die das Steuersignal geschickt wird und welche in Strahlrichtung vor der Reichweitenanpassungseinrichtung (2,109) angeordnet ist, zum Ablenken des Ionenstrahls. (10) aus seiner ursprünglichen Strahlachse (11) auf eine zur ursprünglichen Strahlachse (11) geneigte Strahlachse (11a,11b,11c), so dass der Ionenstrahl (10) auf unterschiedliche Bereiche der Reichweitenanpassungseinrichtung (2,109) gelenkt wird, wobei die Reichweitenanpassungseinrichtung (2,109) auf der geneigten Strahlachse (11a,11b,11c) angeordnet ist,
wobei die Ablenkung des Ionenstrahls (10) in Ansprechen auf das Steuersignal (31) mittels der ersten Ablenkeinrichtung (1,103) derart angepasst wird, dass der Ionenstrahl (10) einen entsprechenden Bereich der Reichweitenanpassungseinrichtung (2,109) durchläuft, so dass die Reichweite des Ionenstrahls (10) auf die durch die Bewegung des Zielvolumens (20) in Strahlrichtung veränderte Position (21, 22, 23) des Zielvolumens (20) abgestimmt ist und das Bragg-Maximum in den Bereich des Zielvolumens (20) geschoben wird, derart dass dadurch die Bewegung des zielvolumens (20) in Strahlrichtung kompensiert wird und
- eine steuerbare zweite Ablenkeinrichtung (3,114) zum Ablenken des Ionenstrahls (10), welche in Strahlrichtung nach der Reichweitenanpassungseinrichtung (2,109) angeordnet ist, so dass der Ionenstrahl (10) aus der geneigten Strahlachse (11a,11b,11c) heraus auf eine Strahlachse (11) gelenkt wird, welche parallel und seitlich versetzt zur ursprünglichen Strahlachse (11), auf welcher der Ionenstrahl (10) in die erste Ablenkeinrichtung (1, 103) eintritt, verläuft.

2. Vorrichtung nach Anspruch 1, **dadurch gekennzeichnet, dass**
die Reichweitenanpassungseinrichtung (2,109) in ihrer Ausdehnung quer zur Strahlachse eine Länge von kleiner als 10 cm und eine Breite von kleiner als 5 cm aufweist.

3. Vorrichtung nach einem der vorstehenden Ansprüche **gekennzeichnet durch**
einen Kollimator (4), welcher in Strahlrichtung nach der Reichweitenanpassungseinrichtung (2,109) positioniert ist, zur Begrenzung des Ionenstrahls (10) in zumindest einer Dimension senkrecht zur Strahlachse (11).

4. Vorrichtung nach einem der vorstehenden Ansprüche **gekennzeichnet durch**
eine Rasterscan-Einrichtung (5,5a,5b) zum zweidimensionalen Scannen des Ionenstrahls (10) in der Ebene senkrecht zur Strahlachse (11) zum Abrastern des Zielvolumens (20).

5. Vorrichtung nach vorstehendem Anspruch **dadurch gekennzeichnet, dass**
die Rasterscan-Einrichtung (5a,5b) auf der Strahlachse (11), auf die der Ionenstrahl (10) mittels der zweiten Ablenkeinrichtung (3,114) gelenkt wird, angeordnet ist.

6. Vorrichtung nach einem der vorstehenden Ansprüche 3 bis 5 **gekennzeichnet durch**
einen Impulsfilter, welcher wenigstens **durch** die zweite Ablenkeinrichtung (3,114) und den Kollimator (4) gebildet ist.

7. Anlage zur therapeutischen Bestrahlung mit Ionenstrahlen umfassend
- einen Beschleuniger zum Erzeugen des Ionenstrahls und
- die Vorrichtung zur Kompensation der Bewegung des zielvolumens (20) in Strahlrichtung, indem die Reichweite des Ionenstrahls (10) an die Bewegung des Zielvolumens (20) angepasst wird nach einem der vorstehenden Ansprüche.

8. Verfahren zur Bestrahlung eines Phantomtargets zur Bestimmung und/oder Verifikation von Verfahrensparametern, umfassend die folgenden Verfahrensschritte:
- Bereitstellen des Ionenstrahls (10),
- Feststellen einer Referenzposition (21, 22, 23) eines zu bestrahlenden zielvolumens (20) eines Phantomtargets,
- Einstellen der Reichweite des Ionenstrahl (10) derart, dass diese an die Referenzposition des Zielvolumens (20) angepasst wird, so dass das Bragg-Maximum im Bereich des Zielvolumens (20) liegt,
- Nachweisen einer Änderung der Referenzposition (21, 22, 23) durch Bewegung des Zielvolumens (20),
- Ablenken des Ionenstrahls (10) durch Einstellen eines angelegten Feldes einer ersten Ablenkeinrichtung (1, 103) aus seiner ursprünglichen Strahlachse (11) auf eine zur ursprünglichen Strahlachse (11) geneigten Strahlachse (11a, 11b, 11c), so dass der Ionenstrahl (10) auf eine Stelle einer Reichweitenanpassungseinrichtung (2), welche in Strahlrichtung vor dem Zielvolumen (20) angeordnet ist, geführt wird, so dass der Ionenstrahl (10) beim Durchlaufen der Reichweitenanpassungseinrichtung (2) an dieser Stelle einen Energieverlust erfährt, welcher derart angepasst ist, dass die Positionsänderung des Zielvolumens (20) durch die Anpassung der Reichweite des Ionenstrahls (10), indem das Bragg-Maximum in den Bereich des Zielvolumens (20) geschoben wird, kompensiert wird,
wobei die Reichweitenanpassungseinrichtung (2,109) auf der geneigten Strahlachse (11a,11b,11c) angeordnet ist,
wobei der Ionenstrahl (10) nach der Reichweitenanpassung aus der geneigten Strahlachse (11a,11b,11c) heraus auf eine Strahlachse (11) gelenkt wird, welche parallel und seitlich versetzt zur ursprünglichen Strahlachse (11), auf welcher der Ionenstrahl (10) in die erste Ablenkeinrichtung (1, 103) eintritt, verläuft.

9. Verfahren nach Anspruch 8, **dadurch gekennzeichnet, dass** der Ionenatrahl (10) auf die Strahlachse (11) gelenkt wird, auf welcher eine Rasterscan-Einrichtung (5,5a,5b) angeordnet ist, so dass die laterale Lage des Ionenstrahls (10) in einer Ebene senkrecht zur Strahlachse (11) zum Abrastern des zielvolumens (20) gezielt variierbar ist.

10. Verfahren nach Anspruch 8 oder 9, **dadurch gekennzeichnet, dass**
Anteile, welche eine nicht angepasste Reichweite aufweisen, aus dem Ionenstrahl (10) gefiltert werden und/oder dass der Ionenstrahl (10) nach der Reichweitenanpassung und vor dem Auftreffen auf dem Zielvolumen (20) wenigstens einmal in zumindest einer zur Strahlachse (11) lateralen Dimension kollimiert wird.

## Claims

1. Device for compensating the movement of a target volume (20) in the beam direction during the therapeutic treatment of a patient by irradiating ions onto the target volume (20), by virtue of the reach of the ion beam (10) being adapted to the movement of the target volume (20), comprising
- a reach adaptation apparatus (2, 109) for the ion beam (10), in which the ion beam (10) experiences different energy losses when passing through different regions of the reach adaptation apparatus (2, 109),
- a sensor (6) for capturing the position (21, 22, 23) of the target volume (20), which sensor provides information (30) describing the position (21, 22, 23),
- a regulation apparatus (7), which receives the information (30) from the sensor (6) and generates a control signal (31) correlated to the position (21, 22, 23) of the target volume (20), and
- a controllable first deflection apparatus (1, 103), to which the control signal is sent and which is arranged upstream of the reach adaptation apparatus (2, 109) in the beam direction, for deflecting the ion beam (10) from the original beam axis (11) thereof to a beam axis (11a, 11b, 11c) tilted with respect to the original beam axis (11), such that the ion beam (10) is deflected to different regions of the reach adaptation apparatus (2, 109), wherein the reach adaptation apparatus (2, 109) is arranged on the tilted beam axis (11a, 11b, 11c),
wherein, as a response to the control signal (31), the deflection of the ion beam (10) is adapted by means of the first deflection apparatus (1, 103) in such a way that the ion beam (10) runs through a corresponding region of the reach adaptation apparatus (2, 109) such that the reach of the ion beam (10) is tuned to the position (21, 22, 23) of the target volume (20) which is modified by the movement of the target volume (20) in the beam direction and the Bragg maximum is shifted into the region of the target volume (20), in such a way that this compensates for the movement of the target volume (20) in the beam direction and
- a controllable second deflection apparatus (3, 114), which is arranged downstream of the reach adaptation apparatus (2, 109) in the beam direction, for deflecting the ion beam (10) such that the ion beam (10) is deflected out of the tilted beam axis (11a, 11b, 11c) and onto a beam axis (11) which extends parallel and laterally offset with respect to the original beam axis (11) along which the ion beam (10) enters the first deflection apparatus (1, 103).

2. Device according to Claim 1, **characterized in that** the reach adaptation apparatus (2, 109) has a length of less than 10 cm and a width of less than 5 cm in its extent perpendicular to the beam axis.

3. Device according to one of the preceding claims, **characterized by**
a collimator (4), which is positioned downstream of the reach adaptation apparatus (2, 109) in the beam direction, for delimiting the ion beam (10) in at least one dimension perpendicular to the beam axis (11).

4. Device according to one of the preceding claims, **characterized by**
a raster scanning apparatus (5, 5a, 5b) for two-dimensional scanning of the ion beam (10) in the plane perpendicular to the beam axis (11) for scanning the target volume (20).

5. Device according to the preceding claim, **characterized in that**
the raster scanning apparatus (5a, 5b) is arranged on the beam axis (11) onto which the ion beam (10) is deflected by means of the second deflection apparatus (3, 114).

6. Device according to one of the preceding Claims 3 to 5, **characterized by**
a pulse filter, which is at least formed by the second deflection apparatus (3, 114) and the collimator (4).

7. Installation for therapeutic irradiation using ion beams, comprising
- an accelerator for generating the ion beam and
- the device for compensating the movement of the target volume (20) in the beam direction by virtue of the reach of the ion beam (10) being adapted to the movement of the target volume (20), according to one of the preceding claims.

8. Method for irradiating a phantom target for determining and/or verifying method parameters, comprising the following method steps:
- providing the ion beam (10),
- determining a reference position (21, 22, 23) of a target volume (20) to be irradiated of a phantom target,
- setting the reach of the ion beam (10) in such a way that it is adapted to the reference position of the target volume (20) such that the Bragg maximum lies in the region of the target volume (20),
- verifying a change in the reference position (21, 22, 23) as result of moving the target volume (20),
- deflecting the ion beam (10) from its original beam axis (11) to a beam axis (11a, 11b, 11c) tilted with respect to the original beam axis (11) by setting an applied field of a first deflection apparatus (1, 103) such that the ion beam (10) is guided onto a position of a reach adaptation apparatus (2), which is arranged upstream of the target volume (20) in the beam direction, in such a way that the ion beam (10) experiences an energy loss when passing through the reach adaptation apparatus (2) at this position, which loss of energy is adapted in such a way that the positional change of the target volume (20) is compensated for by the adaptation of the reach of the ion beam (10) by virtue of the Bragg maximum being shifted into the region of the target volume (20),
wherein the reach adaptation apparatus (2, 109) is arranged on the tilted beam axis (11a, 11b, 11c),
wherein the ion beam (10), after the reach adaptation, is deflected out of the tilted beam axis (11a, 11b, 11c) and onto a beam axis (11) which extends parallel and laterally offset with respect to the original beam axis (11) along which the ion beam (10) enters the first deflection apparatus (1, 103).

9. Method according to Claim 8, **characterized in that** the ion beam (10) is deflected onto the beam axis (11) on which a raster scanning apparatus (5, 5a, 5b) is arranged, such that the lateral position of the ion beam (10) in a plane perpendicular to the beam axis (11) can be varied in a targeted fashion for scanning the target volume (20).

10. Method according to Claim 8 or 9, **characterized in that** components which do not have an adapted reach are filtered out of the ion beam (10) and/or **in that** the ion beam (10) is collimated at least once in at least one dimension lateral to the beam axis (11) after the reach adaptation and prior to being incident on the target volume (20).

## Revendications

1. Dispositif de compensation du mouvement d'un volume cible (20) dans la direction du faisceau lors du traitement thérapeutique d'un patient par irradiation du volume cible (20) avec des ions, par adaptation de la portée du faisceau d'ions (10) au mouvement du volume cible (20), comprenant
- un dispositif d'adaptation de portée (2, 109) pour le faisceau d'ions (10), dans lequel le faisceau d'ions (10) subit une perte d'énergie variable lorsqu'il traverse des zones différentes du dispositif d'adaptation de portée (2, 109),
- un capteur (6) destiné à détecter la position (21, 22, 23) du volume cible (20), qui fournit une information (30) décrivant la position (21, 22, 23),
- un dispositif de régulation (7) qui reçoit l'information (30) du capteur (6) et produit un signal de commande (31) en corrélation avec la position (21, 22, 23) du volume cible (20), et
- un premier dispositif de déviation (1, 103) réglable, auquel est transmis le signal de commande et qui est placé avant le dispositif d'adaptation de portée (2, 109), vu dans la direction du faisceau, pour dévier le faisceau d'ions (10) de son axe de faisceau (11) d'origine, sur un axe de faisceau (11a, 11b, 11c) incliné par rapport à l'axe de faisceau (11) d'origine, de sorte que le faisceau d'ions (10) est dévié sur des zones différentes du dispositif d'adaptation de portée (2, 109), le dispositif d'adaptation de portée (2, 109) étant placé sur l'axe de faisceau incliné (11a, 11b, 11c),
la déviation du faisceau d'ions (10) étant adaptée, en réaction au signal de commande (31), au moyen du premier dispositif de déviation (1, 103), de manière à ce que le faisceau d'ions (10) passe dans une zone correspondante du dispositif d'adaptation de portée (2, 109), de sorte que la portée du faisceau d'ions (10) est adaptée à la position (21, 22, 23) du volume cible (20) modifiée par le mouvement du volume cible (20) dans la direction du faisceau, et le maximum de Bragg est déplacé dans la zone du volume cible (20), de façon à compenser ainsi le mouvement du volume cible (20) dans la direction du faisceau, et
- un deuxième dispositif de déviation (3, 114) réglable pour dévier le faisceau d'ions (10), qui est placé après le dispositif, d'adaptation de portée (2, 109), vu dans la direction du faisceau, de sorte que le faisceau d'ions (10) est dévié de l'axe de faisceau incliné (11a, 11b, 11c), sur un axe de faisceau (11) qui est parallèle et décalé latéralement par rapport à l'axe de faisceau (11) d'origine suivant lequel le faisceau d'ions (10) entre dans le premier dispositif de déviation (1, 103).

2. Dispositif selon la revendication 1, **caractérisé en ce que** le dispositif d'adaptation de portée (2, 109), dans sa dimension transversalement à l'axe de faisceau, présente une longueur inférieure à 10 cm et une largeur inférieure à 5 cm.

3. Dispositif selon l'une des revendications précédentes, **caractérisé en ce qu'**il comprend un collimateur (4) qui, vu dans la direction du faisceau, est positionné après le dispositif d'adaptation de portée (2, 109) pour limiter le faisceau d'ions (10) dans au moins une dimension perpendiculaire à l'axe de faisceau (11).

4. Dispositif selon l'une des revendications précédentes, **caractérisé en ce qu'**il comprend un dispositif de balayage ligne par ligne (5, 5a, 5b) pour le balayage en deux dimensions du faisceau d'ions (10) dans le plan perpendiculaire à l'axe de faisceau (11), en vue du balayage du volume cible (20).

5. Dispositif selon la revendication précédente, **caractérisé en ce que** le dispositif de balayage ligne par ligne (5a, 5b) est placé sur l'axe de faisceau (11) sur lequel est dévié le faisceau d'ions (10) au moyen du deuxième dispositif de déviation (3, 114).

6. Dispositif selon l'une des revendications précédentes 3 à 5, **caractérisé en ce qu'**il comprend un filtre d'impulsions qui est constitué au moins par le deuxième dispositif de déviation (3, 114) et le collimateur (4).

7. Installation de traitement thérapeutique avec des faisceaux d'ions, comprenant
- un accélérateur pour produire le faisceau d'ions, et
- le dispositif de compensation du mouvement du volume cible (20) dans la direction de faisceau, par adaptation de la portée du faisceau d'ions (10) au mouvement du volume cible (20), selon l'une des revendications précédentes.

8. Procédé d'irradiation d'une cible fantôme pour la détermination et/ou la vérification de paramètres de procédés, comprenant les étapes de procédé suivantes:
- mise à disposition du faisceau d'ions (10),
- détermination d'une position de référence (21, 22, 23) d'un volume cible (20) à irradier d'une cible fantôme,
- réglage de la portée du faisceau d'ions (10), de manière à ce que celle-ci soit adaptée à la position de référence du volume cible (20), de sorte que le maximum de Bragg se situe dans la région du volume cible (20),
- détection d'une modification de la position de référence (21, 22, 23) du fait du mouvement du volume cible (20),
- déviation du faisceau d'ions (10), par réglage d'un champ appliqué d'un premier dispositif de déviation (1, 103), de son axe de faisceau (11) d'origine, sur un axe de faisceau (11a, 11b, 11c) incliné par rapport à l'axe de faisceau (11) d'origine, de sorte que le faisceau d'ions (10) est guidé sur un endroit d'un dispositif d'adaptation de portée (2) qui se situe avant le volume cible (20), vu dans la direction du faisceau, de sorte que le faisceau d'ions (10), lorsqu'il passe dans le dispositif d'adaptation de portée (2), subit à cet endroit une perte d'énergie qui est adaptée de manière telle que le changement de position du volume cible (20) soit compensé par l'adaptation de la portée du faisceau d'ions (10), par le fait que le maximum de Bragg est déplacé pour se trouver dans la région du volume cible (20),
le dispositif d'adaptation de portée (2, 109) étant placé sur l'axe de faisceau incliné (11a, 11b, 11c),
le faisceau d'ions (10), une fois l'adaptation de portée effectuée, étant dévié de l'axe de faisceau incliné (11a, 11b, 11c), sur un axe de faisceau (11) qui est parallèle et décalé latéralement par rapport à l'axe de faisceau (11) d'origine suivant lequel le faisceau d'ions (10) entre dans le premier dispositif de déviation (1, 103).

9. Procédé selon la revendication 8, **caractérisé en ce que** le faisceau d'ions (10) est dévié sur l'axe de faisceau (11) sur lequel est placé un dispositif de balayage ligne par ligne (5, 5a, 5b), de sorte que la position latérale du faisceau d'ions (10) peut être modifiée de façon ciblée dans un plan perpendiculaire à l'axe de faisceau (11), en vue de balayer le volume cible (20).

10. Procédé selon la revendication 8 ou 9, **caractérisé en ce que** des fractions qui présentent une portée non adaptée sont éliminées du faisceau d'ions (10) par filtrage, et/ou **en ce que** le faisceau d'ions (10), après l'adaptation de portée et avant l'impact sur le volume cible (20), est soumis au moins une fois à une collimation dans au moins une dimension latérale par rapport à l'axe de faisceau (11).
